# EUROPEAN PATENT SPECIFICATION

(11) **EP 1 825 862 B1**
(45) Date of publication and mention of the grant of the patent: **04.03.2009**
(21) Application number: 06255906.7
(22) Date of filing: 17.11.2006
(51) Int. Cl.: A61K 38/18, A61P 25/00

(54) **Method for treating nerve injury and vector construct for the same**
Methode und Vektor zur Behandlung einer Nervenverletzung
Méthode de traitement et vecteur pour le traitement d'une lésion nerveuse

(30) Priority: 09.12.2005 US 298821
(43) Date of publication of application: 29.08.2007
(73) Proprietor: Henrich, Cheng, 11217 Taipei (TW)
(72) Inventor: Cheng, Henrich, Taipei, Taiwan 11217 (TW); Chang, Pei-Teh, Taiwan 11217 (TW)
(74) Representative: Wright, Simon Mark

(56) References cited:
- EP-A1- 0 488 528
- WO-A-92/11360
- WO-A-20/05037211
- US-A- 6 156 303
- DATABASE MEDLINE [Online] US NATIONAL LIBRARY OF MEDICINE (NLM), BETHESDA, MD, US; June 1989 (1989-06), CORDEIRO P G ET AL: "Acidic fibroblast growth factor enhances peripheral nerve regeneration in vivo." XP002442572 Database accession no. NLM2727148 & PLASTIC AND RECONSTRUCTIVE SURGERY JUN 1989, vol. 83, no. 6, June 1989 (1989-06), pages 1013-1019 ; di, ISSN: 0032-1052

## Description

### BACKGROUND OF THE INVENTION

Disclosed herein is a method for treating nerve injury or regenerating neurons and a vector construct for the same. The invention further relates to the use of the vector for treating nerve injury or regenerating neurons.

Neuronal regeneration and restoration of neural connectivity within denervated tissues may be desirable events following acute or chronic nervous system injury resulting from physical transaction or trauma, contusion or compression or surgical lesion, vascular pharmacologic insults including hemorrhagic or ischemic damage, or from neurodegenerative or other neurological diseases.

Typically, efforts to repair the injured nerve following nervous system injury have been directed to performing surgical nerve suture and nerve grafting for the patient. Other prospects of nerve injury treatment include transplanting new nerve cells and supporting cells, delivering proteins that stimulate regeneration by the cells already in the spinal cord, and strategies to reduce inhibition of regeneration. Some studies have found injured spinal cord in rats was repaired upon direct administration of a nerve repairing agent at or near lesion sites (Cheng et al., 1996, Science 273: 510-513).

In particular, a published US patent application Publication No. US20040267289 disclosed a nerve root repair method which involved bringing a portion of the peripheral nervous system of a vertebrate close to a portion of the central or peripheral nervous system of the vertebrate, and applying to a gap between the two portions a fibrin glue mixture containing a growth factor, fibrinogen and other essential elements, so that a functional connection is formed between the portion of the peripheral nervous system and the portion of the central or peripheral nervous system of the vertebrate.

### BRIEF SUMMARY OF THE INVENTION

In one aspect the invention provides use of a gene coding for an acid fibroblast growth factor (aFGF) for the manufacture of a medicament for treating a nerve injury or regenerating a neuron, wherein the gene is delivered by a viral vector and wherein the viral vector comprises at least one of an adeno-associated virus (AAV) gene and an adenovirus gene.

It is another aspect of the invention to provide a vector construct for treating nerve injury, which comprises a viral vector carrying a gene coding for an aFGFwherein the viral vector comprises at least one of an adeno-associated virus (AAV) gene and an adenovirus gene.

Also described herein is a method for regenerating a neuron which comprises steps of delivering a gene coding for an aFGF to a neuron; and allowing the gene to be expressed in the neuron.

In still another aspect the invention provides a vector construct for regenerating a neuron, which comprises a viral vector carrying a gene coding for an aFGFwherein the viral vector comprises at least one of an adeno-associated virus (AAV) gene and an adenovirus gene.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments which are presently preferred. It should be understood, however, that the invention is not limited to the precise cloning vectors and regulatory genes shown.

In the drawings:

Fig. 1A is a vector map of an A2 clone vector carrying human aFGF (h-aFGF) gene of SEQ ID NO: 1;

Fig. 1B is a vector map of an A4 clone vector carrying human aFGF gene of SEQ ID NO: 1 and rep gene;

Fig. 1C is a vector map of an adeno-associated virus (AAV)-phage hybrid phagemid vector carrying human aFGF (h-aFGF) gene of SEQ ID NO: 1, elongation factor (EF), poly-A and WPRE (woodchuck hepatitis virus posttranscriptional regulatory element) sequences;

Fig. 1D is a vector map of an AAV-phage helper vector carrying the rep-2 and cap-2 genes;

Fig. 2A is a bar graph representing a neuronal differentiation of PC 12 cells at various AAV-aFGF titers over a time course;

Fig. 2B is a bar graph representing a cell proliferation of PC12 cells at various AAV-aFGF titers;

Figs. 3A and 3B are bar graphs representing quantitative analysis of *in vitro* expression of AAV-aFGF, wherein Fig. 3A depicts the amount of aFGF expressed in PC12 cells by western blotting analysis over a time course, and Fig. 3B depicts the amount of aFGF expressed in the extracellular area by western blotting analysis;

Fig. 4 is a microscopic image of stable transfectant expressing aFGF in the ventral horn area of a rat's spinal cord;

Figs. 5A-5C are bar graphs representing quantitative western blot analyses on *in vivo* expression of AAV-aFGF, wherein Fig. 5A depicts the absolute amount of aFGF mRNA expressed in the spinal cord tissue at various AAV-aFGF titers, Fig. 5B depicts the calibrated amount of aFGF expressed in the spinal cord tissue at various AAV-aFGF titers, and Fig. 5C depicts the calibrated amount of purified aFGF expressed in the spinal cord tissue at various AAV-aFGF titers; and

Figs. 6A and 6B are bar graphs representing effect of aFGF gene transduction on behavioral assessment over a time course.

### DEFINITIONS

To facilitate the understanding of the invention, a number of terms are defined below.

The article "a" and "an" are used herein to refer to one or more than one (i.e. to at least one) of the grammatical object of the article. By way of example, "an element" means one element or more than one element.

A "vector" is a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "vector" includes an autonomously replicating plasmid or a virus. The term should also be construed to include non-plasmid and non-viral compounds which facilitate transfer of nucleic acid into cells, such as, for example, polylysine compounds, liposomes, and the like. Examples of viral vectors include, but are not limited to, adenoviral vectors, adeno-associated virus vectors, retroviral vectors, and the like.

As used herein, the term "adeno-associated virus (AAV) vector" refers to a vector derived from an adeno-associated virus serotype, including without limitation, AAV-1, AAV-2, AAV-3, AAV-4, AAV-5, AAVX7, etc.

AAV vectors can also include transcription sequences such as polyadenylation sites, as well as selectable markers or reporter genes, enhancer sequences, and other control elements which allow for induction of transcription.

As used herein, the term "AAV virion" refers to a complete virus particle. An AAV virion may be a wild type AAV virus particle (comprising a linear, single-stranded AAV nucleic acid genome associated with an AAV capsid, i.e., a protein coat), or a recombinant AAV virus particle. In this regard, single-stranded AAV nucleic acid molecules (either sense/coding strand or the antisense/anticoding strand as those terms are generally defined) can be packaged into an AAV virion; both the sense and the antisense strands are equally infectious.

A "gene" refers to a polynucleotide containing at least one open reading frame that is capable of encoding a particular polypeptide or protein after being transcribed or translated. Any of the polynucleotide sequences described herein may be used to identify larger fragments or full-length coding sequences of the genes with which they are associated. Methods of isolating larger fragment sequences are known to those of ordinary skill in the art.

A cDNA library contains only complementary DNA molecules synthesized from mRNA molecules in a cell.

A "primer" refers to a polynucleotide that is capable of specifically hybridizing to a designated polynucleotide template and providing a point of initiation for synthesis of a complementary polynucleotide. Such synthesis occurs when the polynucleotide primer is placed under conditions in which synthesis is induced, i.e., in the presence of nucleotides, a complementary polynucleotide template, and an agent for polymerization such as DNA polymerase. A primer is typically single-stranded, but may be double-stranded. Primers are typically deoxyribonucleic acids, but a wide variety of synthetic and naturally occurring primers are useful for many applications. A primer is complementary to the template to which it is designed to hybridize to serve as a site for the initiation of synthesis, but need not reflect the exact sequence of the template. In such a case, specific hybridization of the primer to the template depends on the stringency of the hybridization conditions. Primers can be labeled with e.g. chromogenic, radioactive, or fluorescent moieties and used as detectable moieties.

The term "oligonucleotide" typically refers to a short polynucleotide, generally no greater than about 50 nucleotides. It will be understood that when a nucleotide sequence is represented by a DNA sequence (i.e. A, T, G, C), this also includes an RNA sequence (i.e., A, U, G, C) in which "U" replaces "T".

As used herein, a "sequencing primer" is an oligonucleotide primer which is complementary to at least a portion of a polynucleotide and which can be elongated by a DNA or RNA polymerizing enzyme such as DNA polymerase, whereby binding of the sequencing primer to the polynucleotide and elongation of the primer using methods well known in the art yields an oligonucleotide transcript which is complementary to at least a part of the polynucleotide.

As used herein, the term "reporter gene" means a gene, the expression of which can be detected using a known method. By way of example, the *Escherichia coli* (*E coli*) *lacZ* gene may be used as a reporter gene in a medium because expression of the *lacZ* gene can be detected using known methods by adding the chromogenic substrate *o*-nitrophenyl-β-galactoside to the medium (Gerhardt et al., 1994, Method for General and Molecular Bacteriology, American Society for Microbiology, Washington, DC, p. 574).

As used herein, the term "promoter/regulatory sequence" means a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

A restriction site is a portion of a double-stranded nucleic acid which is recognized by a restriction endonuclease. A portion of a double-stranded acid is "recognized" by a restriction endonuclease if the endonuclease is capable of cleaving both strands of the nucleic acid at the portion when the nucleic acid and the endonuclease are contacted.

The term "transfection" refers to a method by which genetic material, such as nucleic acid may be put into a cultured cell.

An "infection" by medical dictionary definition is the detrimental colonization of a host organism by an infecting organism or pathogen including bacteria, parasites, fungi, viruses, prions, and viroids which multiplies at the expense of the host organism. In one preferable embodiment of the invention, the term "infection" is a viral infection that is not detrimental, by which viral genetic material can be introduced into appropriate host cells, and which the virus can recognize by means of proteins on its outermost surface. The virus nucleic acid uses the host machinery within the cells to make copies of viral nucleic acid as well as enzymes needed by the virus, and enveloping proteins of the virus.

As used herein, the term "nerve injury" means an acute or chronic injury to or adverse condition of a nerve resulting from physical transaction or trauma, contusion or compression or surgical lesion, vascular pharmacologic insults including hemorrhagic or ischemic damage, or from neurodegenerative or any other neurological disease, or any other factor causing the injury to or adverse condition of the nerve.

A "polypeptide" refers to a polymer composed of amino acid residues, related naturally occurring structural variants, and synthetic non-natually occurring analogs thereof linked via peptide bonds, related natually occurring structural variants, and synthetic non-natually occurring analogs thereof. Synthetic polypeptides can be synthesized, for example, using an automated polypeptide synthesizer. Convnetional notation is used herein to portray polypeptide sequences: the left-handed end of a polypeptide sequence is the amino-terminus; the right-hand end of a polypeptide sequence is the carboxyl-terminus.

### DETAILED DESCRIPTION OF THE INVENTION

The present invention relates to treating nerve injury. The invention involves delivering a gene coding for acidic fibroblast growth factor (aFGF) to a nerve injury site. The aFGF gene is then allowed to be expressed at the nerve injury site, where the aFGF gene is either transcribed into aFGF mRNA or further translated into an aFGF polypeptide. The nerve injury site may be a nerve organ, nerve tissue, a neuronal cell system transducible with the vector, or a site at which neuronal cells or processes thereof may reside, including but not limited to neuronal axonal projection tracts.

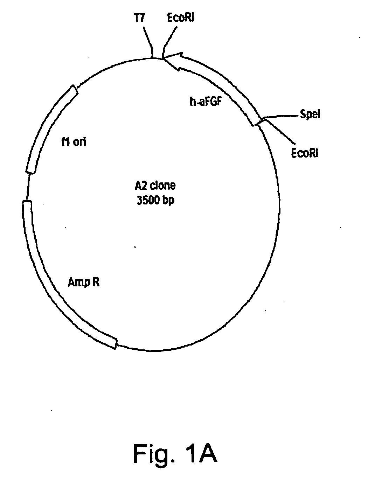

In one preferred embodiment of the invention, the aFGF gene has, comprises or consists of a DNA sequence of SEQ ID NO: 1. Referring to Figs. 1A through to 1D, a vector is constructed to comprise the aFGF gene of SEQ ID NO: 1. The vector is a viral vector comprising a viral genome for packaging the vector into viral particles. The viral genome may be a genome of a recombinant virus, such as a recombinant adenovirus, adeno-associated virus (AAV), herpes virus, pox virus or retrovirus. According to the invention, any viral vector comprising at least one of an adeno-associated virus (AAV) gene and adenovirus gene may be used to integrate the aFGF gene into the host cell genome. The vector construct preferably also comprises a reporter gene, preferably a bacterial gene such as *lacZ* cDNA. Methods which are well known to those skilled in the art can be used to construct expression vectors containing the aFGF coding sequence: it may be operatively associated with appropriate transcriptional and/or translational control signals. These methods include *in vitro* recombination DNA techniques described in Sambrook, et al., 1992, Molecular Cloning, A Laboratory Manual, Cold Spring Harbor Laboratory, N.Y.

In accordance with another preferred embodiment of the invention, the vector may be constructed to comprise a gene coding for an aFGF having, comprising or consisting of an amino acid sequence having at least 70%, at least 75%, at least 80%, at least 85%, at least 90%, at least 95%, at least 98% or at least 99% identity with SEQ ID NO: 2. In accordance with a more preferred embodiment of the invention, the vector may be constructed to comprise a gene coding for an aFGF having, comprising or consisting of an amino acid sequence that is identical to SEQ ID NO: 2. The aFGF of SEQ ID NO: 2 includes aFGF polypeptides of SEQ ID NO: 2 folded into different forms and protein configurations.

In accordance with a further embodiment, the vector comprises aFGF cDNA obtained from a human brain (cDNA library). However, the vector construct should not be limited as such, and the aFGF gene of other species may also be used for the vector construction depending on the type of the nerve injury treatment or neuronal regeneration desired.

Also described herein is a vector that is at least one of a plasmid and a bacteriophage, for example, an AAV plasmid such as pSub201 first disclosed by Samulski et al. (Journal of Virology, 1987, Vol. 61, No. 10, pp. 3096-3101). This vector can contain all of the adeno-associated virus type 2 (AAV-2) wild-type coding regions and cis acting terminal repeats cloned into a plasmid backbone. This vector is ideal for cloning, as it was engineered in such a way that restriction digest with restriction enzyme Xba I allowed one to remove the AAV coding region while leaving the AAV terminal repeats intact in the plasmid backbone.

The vector can further contain a helper plasmid pDG (Grimm et al., 1998, Human Gene Therapy 9:2745-2760), e.g. having both the AAV genes (rep and cap) and helper genes required for AAV propagation so as to generate recombinant AAV. This plasmid can supply the AAV and adenoviral gene functions required for amplification and packaging of the AAV vector.

Therefore, in packaging of AAV particles, the cells were cotransfected with the pSub201 plasmid carrying aFGF cDNA and the pDG helper plasmid for AAV packaging preferably by calcium phosphate plasmid transfection. The mature virion was packaged in the host cells. In the present embodiment, the host cell may be a HEK293 cell.

With respect to treating nerve injury, other modes of delivering the vector comprising the aFGF gene to the nerve injury site includes infecting the nerve injury site with a viral vector having an aFGF gene; injecting viral particles that carry the aFGF gene to the nerve injury site; and transfecting the aFGF gene into the nerve injury site. The aFGF gene can be transfected into the nerve injury site by cell transfection methods, such as electroporation, microinjection, liposomal transfection reagent, polyethylenimine (PEI), effectene and activated-dendrimers, all as known by those skilled in the art.

Alternatively, treating the nerve injury comprises infecting a nerve injury site with a viral particle having an aFGF gene so that the aFGF gene is expressed at the nerve injury site. The nerve injury site is infected with the viral particle according to a lysogenic pathway where the aFGF gene is integrated into a specific site in the genome of cells in the nerve injury site and replicates as the cells replicate. The viral vector may be a virus containing vector for packaging into viral particles that carry the aFGF cDNA. Preferably, the viral vector is a pPAM-AAV plasmid containing the cDNA of human aFGF and/or bacterial *LacZ.*

Another embodiment of the invention relates to regenerating neuron is also provided. This involves delivering a vector comprising an aFGF gene to a neuron. The aFGF gene can be delivered to the neuron in any manner discussed above with reference to treating nerve injury. The aFGF gene is allowed to be expressed in the neuron, where the aFGF gene is then transcribed and translated in such a way as to produce an aFGF polypeptide in the neuron. The neuron that expresses the aFGF may also include astrocytes, oligodendrocytes, neuroglial cells, choroids plexus cells, ependymal cells, meningeal cells, Schwann cells, fibroblasts, microglial cells, cells in the spinal cord tissue or any cell line of neuronal origin transducible with the above-discussed vector construct, such as (pheochromocytoma) PC 12 cells.

In the preferred embodiment of the invention, the aFGF gene may be a gene having, comprising or consisting of a DNA sequence having at least 70%, at least 75%, at least 80%, at least 85%, at least 90%, at least 95%, at least 98% or at least 99% identity with SEQ ID NO: 1, or a gene coding for an aFGF having, comprising or consisting an amino acid sequence having at least 70%, at least 75%, at least 80%, at least 85%, at least 90%, at least 95%, at least 98% or at least 99% identity with SEQ ID NO: 2. In a more preferred embodiment of the invention, the aFGF gene may be a gene having, comprising or consisting of a DNA sequence that is identical to SEQ ID NO: 1, or the gene coding for an aFGF having, comprising or consisting an amino acid sequence that is identical to SEQ ID NO: 2. The aFGF gene may be operatively associated with one or more different promoter and/or enhancer element(s), which may include but need not be limited to a promoter, an enhancer, a transcription factor binding site and other gene expression regulatory sequences, such as elongation factor (EF), a polyadenylation (PolyA) sequence, a long terminal repeat (LTR), an antibiotic resistance gene and so on. The expression elements of these vectors may vary in their strength and specificities. Depending on the host/vector system utilized, any one of a number of suitable transcription and translation elements may be used. The promoter may be in the form of the promoter which is naturally associated with the gene of interest. Alternatively, the DNA may be positioned under the control of a recombinant or heterologous promoter, i.e., a promoter that is not normally associated with that gene. In any event, the promoter is usually included as an "operatively linked" promoter, which refers to the situation of a promoter that initiates transcription of the aFGF gene of SEQ ID NO: 1 in any embodiment described above.

The invention will now be described in further detail with reference to the following specific, non-limiting examples.

Example 1: Construction of pPAM-AAV-plasmid containing full-length human aFGF cDNA and generation of the AAV-aFGF particles

A pPAM-AAV plasmid containing the cDNA of human aFGF and/or bacterial *LacZ* was constructed as follow. First, the human aFGF cDNA was cloned and amplified. The human aFGF cDNA, from human brain cDNA library (Clontech Laboratories, Inc., Mountain View, CA, USA), was subjected to PCR analysis for human aFGF by using Expand High Fidelity PCR system (Roche Diagnostics GmbH, Nonnenwald, Germany) following the conditions: PCR step thirty cycle; 95°C for 30s, 60°C for 60s and 72°C for 30s. For the control vector, the reporter gene of full-length bacterial *lacz* cDNA was amplified by PCR reaction following the conditions: thirty cycle; 95°C for 30s, 55°C for 60s and 72° C for 30s from pDNR-*LacZ* Donor Reporter (Clontech Laboratories, Inc., Mountain View, CA, USA). The primers for aFGF and/or *lacZ* were obtained from MDBio Inc. (Taipei, Taiwan) and their sequences are listed below: Primers for human aFGF (forward sequencing primer site, 5'-ATGGCTGAAGGGGAAATC-3' of SEQ ID NO:3, and reverse sequencing primer site, 5'-TTAATCAGAAGAGACTGGCAGGGG-3' of SEQ ID NO:4); and primers for *LacZ* (forward sequencing primer site, 5'-ATGTCGTTTACTTTGACCAACAAG-3' of SEQ ID NO:5, and reverse sequencing primer site, 5'-CTTTTTTTGACACCAGACCAACTGG-3' of SEQ ID NO:6).

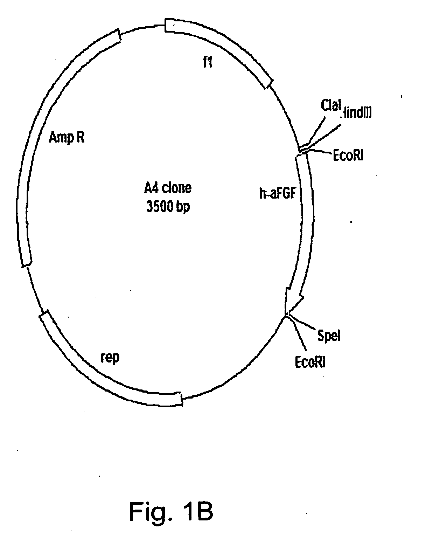

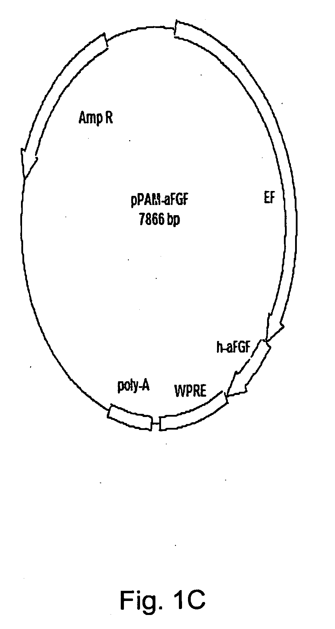

The amplified fragments were cloned directly into a pGEM-T easy TA vector system (Promega Corp., Madison, WI, USA), so that an A2 clone containing cDNA of human aFGF was obtained as shown in Fig. 1A. The A2 clone was then digested with the restriction enzyme EcoRI and eluted, so that an eluted fragment containing human aFGF sequence was ligated with an EcoRI-digested pBluescript-SK plasmid (Stratagene Corp., La Jolla, CA, USA). As a result, the pBluescript -plasmid that contained aFGF sequence was generated and shown as an A4 clone in Fig. 1B after being checked with the precisely orientated sequence from two possible orientations. Subsequently, an AAV plasmid that contained human aFGF cDNA was created by ligation of a smaller fragment from the A4 clone and a plasmid pSub201 co-digested by the restriction enzyme HindIII/XbaI. The AAV plasmid which carried the human aFGF cDNA is shown as pPAM-AAV in Fig. 1C and abbreviated as AAV-aFGF in the subsequent examples. The plasmid pSub201 containing the wild-type (wt) AAV genome and the recombinant helper plasmid pDG were kind gifts of Dr. Hsu Ma (Division of Plastic Surgery, Veterans General Hospital, Taipei, Taiwan) (reference: The Journal of Trauma, March 2003; 54(3): 569-73, "Gene therapy into Human keloid tissue with adeno-associated virus vector").

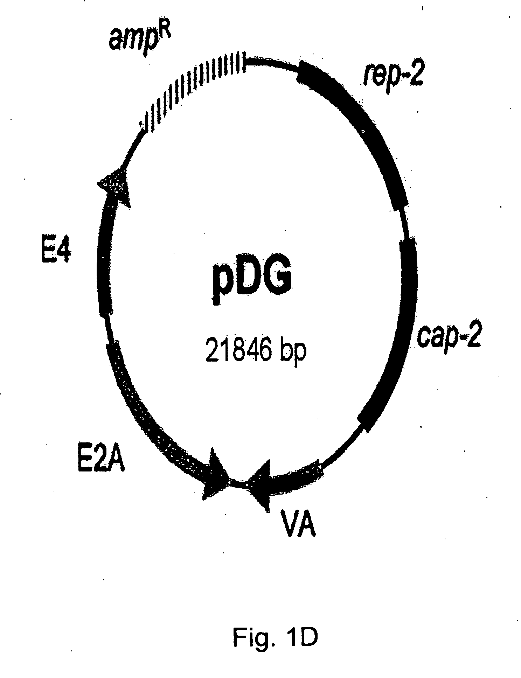

The mature virions were packaged on HEK293 cells (LifeTechnologies Inc., Grand Island, NY, USA) by calcium phosphate plasmid transfection. For packaging of AAV particles, cells were cotransfected with the pPAM-AAV plasmid which carried aFGF cDNA and the pDG helper plasmid which contained the wt AAV and adenovirus genes necessary for AAV packaging as shown in Fig. 1D. The cells were harvested 48 hours post-transfection with rubber policemen, suspended in Opti-MEN solution (Gibco® Invitrogen, Inc., Carlsbad, CA, USA) and sonicated. The cell suspension was then mixed with one-third of its volume of 1,1,2-tri- chloro-trifluoroethane. Further purification of AAV-aFGF particles was done by two rounds of cesium chloride (CsCl) density gradient centrifugation. Next, DNase I treatment of viral preparations was performed to digest unencapsidated DNA, and the supernatants containing the purified AAV-aFGF particles were preserved at -80°C. The titers of the purified AAV-aFGF particles were estimated by sandwich ELISA kits obtained from Progen Biotechnik GmbH (Heidelberg, Germany). Similarly, the preparation of the AAV*-lacZ* particles was performed in accordance with the procedures for preparing the AAV-aFGF particles.

Example 2: *In Vitro* functional assay of recombinant AAV-aFGF in cultured PC12 neurons

Rat pheochromocytoma PC12 cells (ATCC^{®} Number: CRL-1721™) were cultured in medium containing 85% (v/v) of RPMI -1640 (Gibco® Invitrogen, Inc., Carlsbad, CA, USA), 10% (v/v) of heat-inactivated horse serum (HS) and 5% (v/v) of fetal calf serum (FCS) (Biochrom Ltd., Berlin, Germany), 2 mM glutamine, and 25 mM HEPES at pH 7.3 with 100 U/ml penicillin and 100 µg/ml streptomycin. The PC12 cells were maintained in a humidified incubator at 37°C with 5% CO₂. During the analysis, PC 12 cells were cultured in RPMI supplemented with 2 % HS and 1 % FCS. Also, in providing a positive control group, 100 ng/ml of NGF (CytoLab Ltd., Rehovot, Israel) was added to the cell culture for analyzing the differentiation and/or proliferation of PC 12 cells. All other materials were purchased from Sigma Chemical Co. (St. Louis, MO, USA).

In the neuron differentiation analysis, the cells were subcultured into a 6-well plate at density of 5×10⁵ cells/well. To measure neurite outgrowth induced by AAV-aFGF, the cultured medium was replaced by serum-free RPMI -containing control vehicle or different amounts of AAV-aFGF (200, 2000 or 20000 virus particles per cells). After the cells were exposed in AAV-aFGF for 8 hours, the serum-free medium containing virus was then removed and cells were incubated with RPMI supplemented with 2% HS and 1% FCS for another 24 or 48 h. The level of neurite outgrowth was observed and photographed under a microscope (Eclipse® T100™, Nikon Corp., Tokyo, Japan) after the cells were fixed with 4% paraformaldehyde in PBS. The total neurite lengths were measured from at least 100 cells per condition, from triplicate wells and from three independent experiments, and were quantified by using the National Health Institute (NIH) Image software, ImageJ which is available on the NIH website, http://rsb.info.nih.gov/ij/.

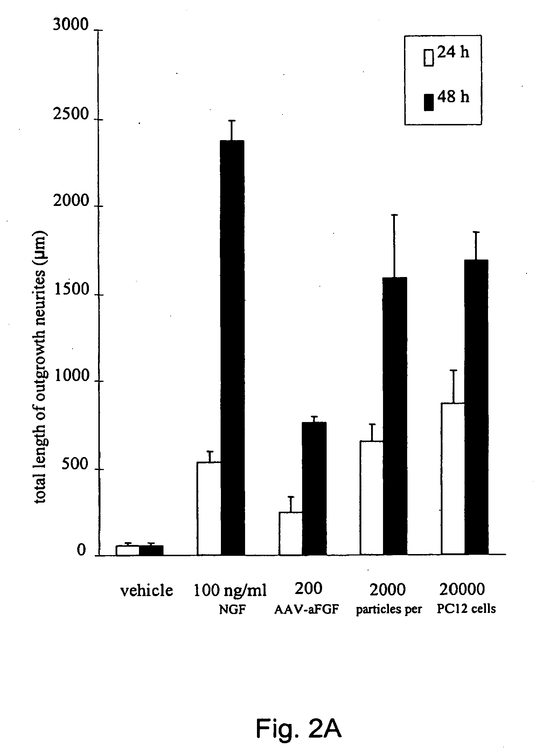

Referring to Fig. 2A, the neurite outgrowth was evident in the PC 12 cells when the cells were treated with AAV-aFGF particles, indicating neuronal differentiation was triggered by AAV-aFGF. The total length of outgrowth neurite (measured in µm) increased when the titer was increased to 200, 2000 and 20000 AAV-aFGF particles. The neuronal differentiation of the PC 12 cells also increased at the specific titer as the incubation period was extended from 24 hours to 48 hours.

For the measurement on the proliferation effect of AAV-aFGF, the PC12 cells were grown at 37°C in RPMI media supplemented with 2% HS and 1% FCS in a 96-well plate at a density of 5x10³ cells/well. The cultured medium was replaced by serum-free RPMI containing control vehicle or different amounts of AAV-aFGF (20, 200, 2000 or 20000 virus particles per cell). After the cells were exposed in AAV-aFGF for 8 hours, the serum-free medium containing virus was then removed. Then the cells were incubated with RPMI supplemented with 2% HS and 1% FCS for another 24 hours. After culturing for 24 hours, cells were washed by warm serum-free RPMI-1640 and added with 0.1 ml of MTT (3-[4,5-dimethyl- thiazol-2-yl] 2,5-diphenyltetrazolium bromide (Promega Corp., San Luis Obispo, CA, USA)) working solution (5 mg/ml MTT in serum-free RPMI-1640) in the wells being assayed. After incubation at 37°C for 3 hours, the working solution was removed and the MTT lysis buffer (20% SDS and 50% DMF in distilled and deionized H₂O) was added to the cells for 6 hours. The absorbance was measured using Tecan® Sunrise^{™} ELISA Reader (Phenix Research Products, Hayward, CA, USA) at a wavelength of 570 nm with background subtraction at a wavelength of 650 nm. The proliferation induced by aFGF was also assayed by tryptan blue exclusion assay using microscopy and also by PI staining with flow cytometry. The cell proliferation percentage of each group was calculated by comparing the measured absorbance of each group with the absorbance (at 570 nm with background subtraction at 650 nm) measured for the control group, which was taken as 100%.

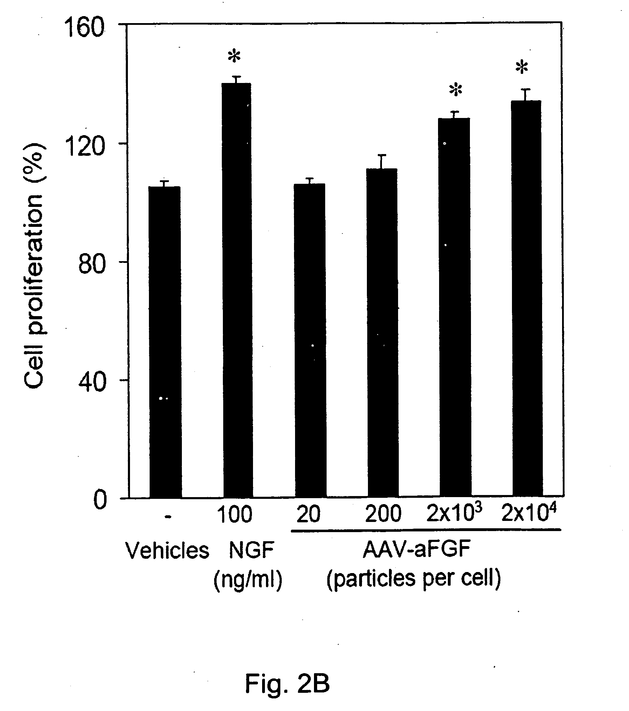

As shown in Fig. 2B, the AAV-aFGF particles resulted in increased proliferation of the PC 12 cells as the dose was increased to 200, 2000 and 20000 AAV-aFGF particles per cell. For instance, the cell proliferation was increased more than 120% when the cells were treated at a dose of 2000 or 20000 virus particles per cells. Thus, the mitogenic effect of AAV-aFGF on PC 12 cells also had a dose-dependent response.

Example 3: Qualitative and quantitative analyses on *in vitro* expression of recombinant AAV-aFGF

PC12 cells were infected with recombinant AAV-aFGF in serum-free RPMI for 8 hours, the cells were then cultured in RPMI medium containing 2% HS and 1%FCS for at least 24 hours in an immunocytochemical analysis (ICC) and cultured in the medium for 24, 48, and 72 hours, respectively, in the western blotting analysis.

In the ICC analysis, the PC12 cells were fixed with methanol/acetone/PBS (V/V/V=1:1:8, in 10 mM MES, pH 6.9, 0.1 mM EGTA and MgCl₂ in 1X PBS) for 25 min at 4°C 24 hours post-AAV infection, and then washed with 1X PBS (containing 0.1 % Tween® 20) twice. The cells were then blocked with 1 % FCS in PBS at room temperature for 2 to about 3 hours. After washing with PBS, the cells were incubated with anti-aFGF polyclonal antibody (Santa Cruz Biotechnology, Inc., Santa Cruz, CA, USA) at room temperature for 2 hours. Next, the cells were washed with PBS and H₂O, and further subjected to a gradient solvent wash of 50 % and 70 % ethanol in PBS, and 100 % ethanol. Then, the cells were observed under the microscope. The cells were found to differentiate since phenotypic change, such as neurite formation, occurred when the cells were treated with 2x10³ or 2x10⁴ AAV-aFGF particles per cell.

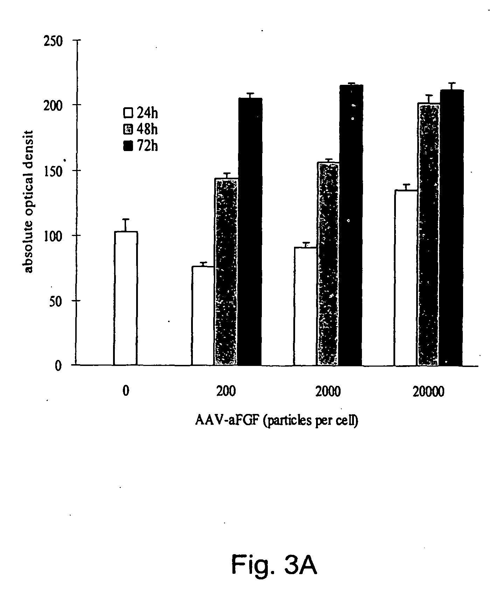

In the western blotting analysis on a protein level of aFGF of SEQ ID NO: 2, the cells were harvested at each time point (24, 48 and 72 hours post AAV-infection). The cells were collected by centrifugation and lysed in a lysis buffer (1% NP-40 containing 30 mM Na₄P₂O₇, 30mM NaF, 1 mM Na₃VO₄, and 1x Protease Inhibitor Cocktail Table (Roche Diagnostics GmbH, Nonnenwald, Germany)). Next, 10 µg of each sample was separated by SDS-PAGE and the expression level of aFGF of SEQ ID NO: 2 was analyzed by using anti-aFGF polyclonal Antibody (Santa Cruz Biotechnology, Inc., Santa Cruz, CA, USA). The SDS-PAGE and immunoblotting were performed according to the method from Conway et al., 1999 Biochemical Journal 337:171-177. Referring to Fig. 3A, the protein level of aFGF of SEQ ID NO: 2 measured in terms of absolute optical density was increased in a time and dose dependent manner since PC 12 cells infected at a dose of 20000 AAV-aFGF particles per cell over 72 hours had higher expression level than the cells infected at lower doses over shorter periods.

A further analysis was performed to determine whether the aFGF of SEQ ID NO: 2 could be secreted into extracellular environment. After PC12 cells were infected with AAV-aFGF (1 µl) in serum-free RPMI medium for 8 hours, cells were then cultured in RPMI medium containing 2% HS and 1% FCS for 24 hours. After incubation for 24 hours, the serum-containing medium was then removed and cells were incubated with serum-free RPMI medium for another 3 hours. Then the serum-free conditional media generated from AAV-infected PC12 cells were harvested. In addition, a positive control group obtained by injecting 1 ng of recombinant human aFGF protein in the conditional medium is provided. These conditional media were dialyzed in 100 RPMI medium (volume 3 times, and 3 hours up to 18 hours) by using dialysis membrane tubing (No. 3: 3.5-kDa cutoff; Spectrum Microgon, Inc., Laguna Hills, CA, USA) and lyophilized and concentrated with a freeze dryer (Labconco Corp., Kansas City, MO, USA). Then the total lyophilized samples were adapted to the SDS-PAGE separation and western blotting analyses as described above.

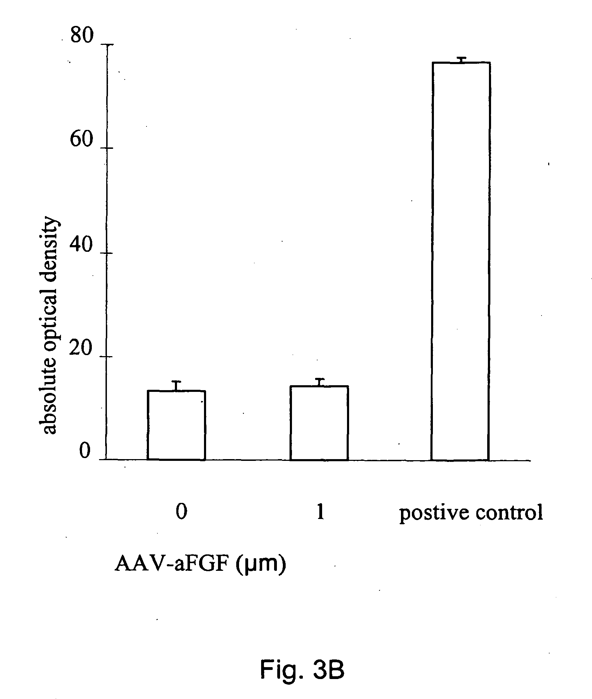

Referring to Fig. 3B, the cells infected with 1 µl of AAV-aFGF showed little or no increase in aFGF expression in the conditional medium, indicating that no aFGF of SEQ ID NO: 2 was secreted or released from cytosol to the extracellular area.

Example 4: Contusive spinal cord injury on SD rats and *in vivo* human aFGF gene transduction into spinal cord of non-injured or injured adult rats by using recombinant AAV-aFGF

Sprague-Dawley (SD) rats were obtained from the Animal Center of National Science Council, Taiwan. Female adult SD rats ranging from 240-280 grams were selected for induction of spinal cord injury (SCI) in this experiment. Animal handling and experimental protocols were carefully be reviewed and approved by the animal studies subcommittee of Veterans Hospital. The female adult SD rats were anesthetized and subjected to laminectomy carried out at the T9-10 levels. SCI was induced by dropping a 10 gram rod from a height of 25-50 mm using a New York University (NYU) weight-drop device, such as NYU impactor after the laminectomy so as to injure the dorsal surfaces of the spinal cord in the contrusion group. The SD rats without SCI were taken as the sham group to contrast with the contrusion group and normal group without any prior treatment and laminectomy. Meanwhile, the extent of injury was quantified with the locomotor rating scale 7, 14, 21, and 28 days post-SCI.

Furthermore, AAV-aFGF was injected to the T8 and T10 segments of the spinal cord in sham + AAV-aFGF group after the laminectomy. The AAV-aFGF was injected to about 1 to about 2 mm rostral and caudal to the epicenter within five minutes after SCI in the contrusion + AAV-aFGF group. For administering the AAV-aFGF, the rats were placed in a stereotaxic frame before injecting 1 µl of recombinant AAV-aFGF into the target sites of the spinal cord through a 5 µl-Hamilton syringe fitted with a 33-gauge beveled needle at an injection rate of 0.5 µl/min. for 2 minutes. One minute after the injection, the needle was retracted 0.5 mm backwards and remained in place for one more minute before slowly being withdrawn away from the spinal cord.

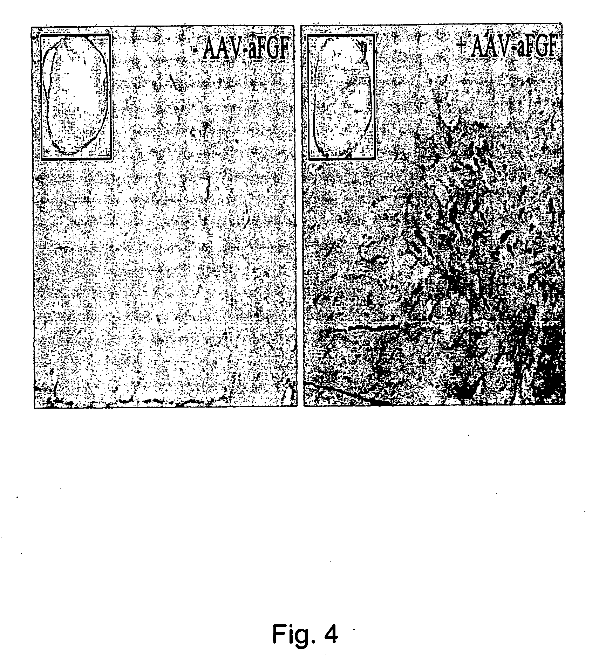

To locate the expression of transgene "human aFGF" on the spinal cord, the spinal cords of the rats were dissected, fixed and cryo-protected; and serial transverse sections were made by cryostat for immunohistological analysis after 7 days. The rats of the sham group were perfused with normal saline and 4% paraformaldehyde in PBS seven days after the surgery. Then spinal cords were removed and fixed for 2 hours with 4% of paraformaldehyde in PBS. After storing in 0.8 M sucrose solution for 2 weeks, the spinal cords were horizontally sectioned into slices each at 10 nm thickness using a cryostat at -20°C. Immunoreactivity of aFGF on the spinal cord section was stained with anti-aFGF monoclonal or polyclonal antibodies (Santa Cruz Biotechnology, Inc., Santa Cruz, CA, USA; and R & D Systems, Inc., Minneapolis, MN, USA) and photographed 14 days after the AAV-infection. Referring to Fig. 4, the aFGF-postive neurons were found in the ventral horn area of the spinal cord.

For analysis of the expression level of aFGF after AAV-aFGF challenge, the spinal cord tissues were immediately removed, dissected into segments or regions and frozen 3 and 7 days post surgery. The total RNA (5µg) isolated from the spinal cord T9 segment with and without AAV-infection using a High Pure® RNA extract isolation kit (Roche Diagnostics GmbH, Nonnenwald, Germany), was subjected to reverse-transcriptase polymerase chain reaction (RT-PCR) analysis to determine human aFGF mRNA level by using the SuperScript one-step RT-PCR system (Invitrogen, Inc.) following the reaction [RT step: 55°C for 30 min, and PCR step thirty cycle; 95°C for 30s, 60°C for 55s and 72°C for 30s]. Primers for Human aFGF: (forward primer, 5'-CGAATTCACTAGTGATTATGG-3' of SEQ ID NO: 7 and reverse primer, 5'-CTGCAGGAATTCGATTTTAATC-3' of SEQ ID NO: 8). Primers for rat actin: (forward primer, 5'-AAGATCCTGACCGAGCGTGG-3' of SEQ ID NO: 9 and reverse primer, 5'-AGCGATGCCTGG- GTACATGG-3' of SEQ ID NO: 10). After the PCR reaction, the cDNA products were separated by 0.5% agarose gel and staining with 50 µM EtBr solution. Then the stained gels were adapted to the Foto/UV-26 system (Fotodyne Incorporated, Hartland, WI, USA) and photographed with black and white instant Pack film (Polaroid Corp., Hertfordshire, England) by using Polaroid® GelCamera™. The quantitation for the level of RT-PCR products was performed by using the NIH released software, ImageJ.

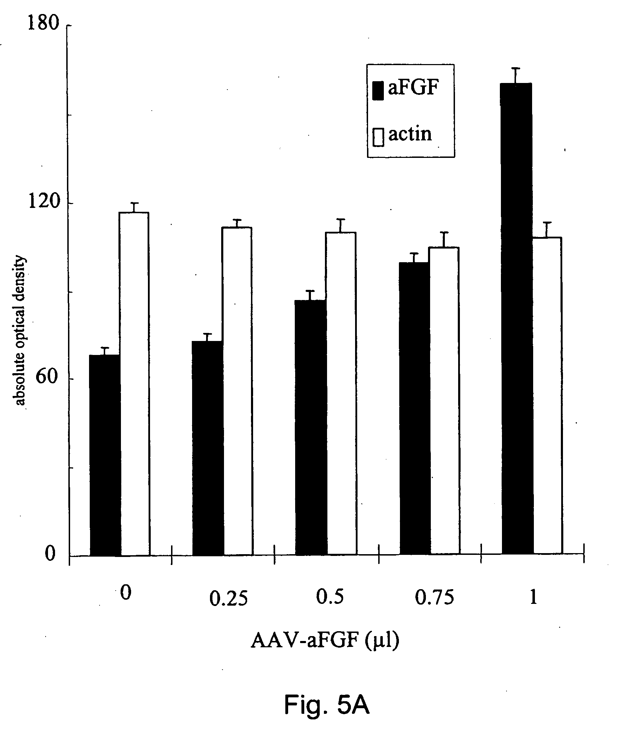

Referring to Fig. 5A, the mRNA level expressed in the spinal cord tissue increased in a dose dependent fashion. However, a dramatic increase in the expression level was evident when the dose of recombinant AAV-aFGF was increased to 1 µl.

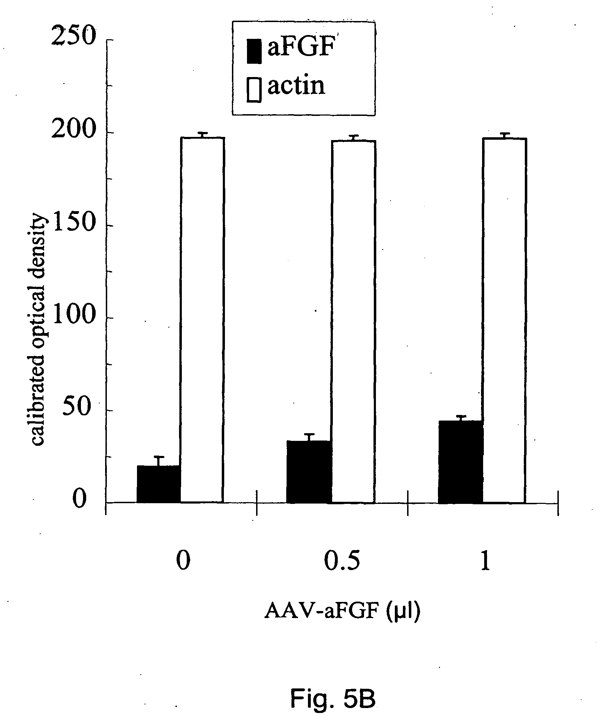

The cells were harvested at each time point (24, 48 and 72 hours) after AAV-infection to analyze the protein level of intracellular aFGF by western blotting analysis. The cytosolic protein was collected from spinal cord without further protein purification procedures. The cells were centrifuged and lysed in the lysis buffer (1% NP-40 containing 30 mM Na₄P₂O₇, 30mM NaF, 1 mM Na₃VO₄, and 1 x Protease Inhibitor Cocktail Table (Roche Diagnostics GmbH, Nonnenwald, Germany). The protein concentrations of tissue samples were assayed using a Bio-Rad® DC kit (Bio-Rad Laboratories, Inc., Hercules, CA, USA). 30 µg of protein from spinal cord tissue at the T9 segment was separated by SDS-PAGE and the expression levels of aFGF and actin were analyzed by western blot with anti-aFGF antibodies and anti-actin antibody, respectively. As shown in Fig. 5B, the spinal cord infected with AAV-aFGF had slightly higher protein expression of aFGF of SEQ ID NO: 2 than the spinal cord not infected with any AAV-aFGF. In addition, 50 µg of tissue protein samples was used to analyze the accurate protein level by using an aFGF ELISA Quantikine kit (R & D Systems Inc., Minneapolis, MN, USA). The level (pg/ml) of aFGF of SEQ ID NO: 2 within 50 µg of protein sample in each group was calculated and is shown in Table 1 below.

**Table 1**

| Day | normal | sham | sham+AAV-aFGF | contusion | contusion+AAV-aFGF |
|---|---|---|---|---|---|
| 3 | ND | 4.48 ± 0.27 | 4.98 ± 0.24 | 2.62 ± 0.28 | 4.10 ± 0.49 |
| 14 | 4.96±0.20 | 4.58 ± 0.37 | 5.22 ± 0.21 | 1.08 ± 0.17 | 5.86 ± 0.35 |

| | | | | | |
|---|---|---|---|---|---|
| Unit: pg/ml | | | | | |

As listed in Table 1, the sham group that received AAV infection generally showed higher aFGF expression (4.98 ± 0.24 or 5.22 ± 0.21 pg/ml) than the sham group without any AAV infection (4.48 ± 0.27 or 4.58 ± 0.37 pg/ml), regardless of whether the treatment period is 3 days or 14 days. The contusion group infected with AAV-aFGF vector showed even more significant increase in aFGF expression (4.10 ± 0.49 or 5.86 ± 0.35 pg/ml) as compared to the contusion group without any treatment.

These *in vivo* tissue samples were also subjected to further protein purification. Each of these samples was directly passed through an Ultrafree®-0.5 Centrifugal filter (Millipore Corp., Billerica, MA, USA), and residual supernatant was collected. The supernatant was separated by a heparin-affinity column (Amersham Pharmacia Biotech, Piscataway, NJ, USA) with binding buffer (10 mM of sodium phosphate buffer, pH 7.0) and was eluted by gradient binding buffer (gradient concentration NaCl ranging from 0.15, 0.5, 0.8 through 1.5 M in 10 mM sodium phosphate buffer, pH 7.0). The eluted protein solution was desalted by passing through a Sephadex® G25 spin column (Amersham Pharmacia Biotech, Piscataway, NJ, USA), and lyophilized/concentrated with a freeze dryer (Labconco Corp., Kansas City, MO, USA). The total lyophilized samples were separated by SDS-PAGE and the expression level of aFGF of SEQ ID NO: 2 was analyzed by western blotting analysis using anti-aFGF antibody and anti-actin antibody, respectively.

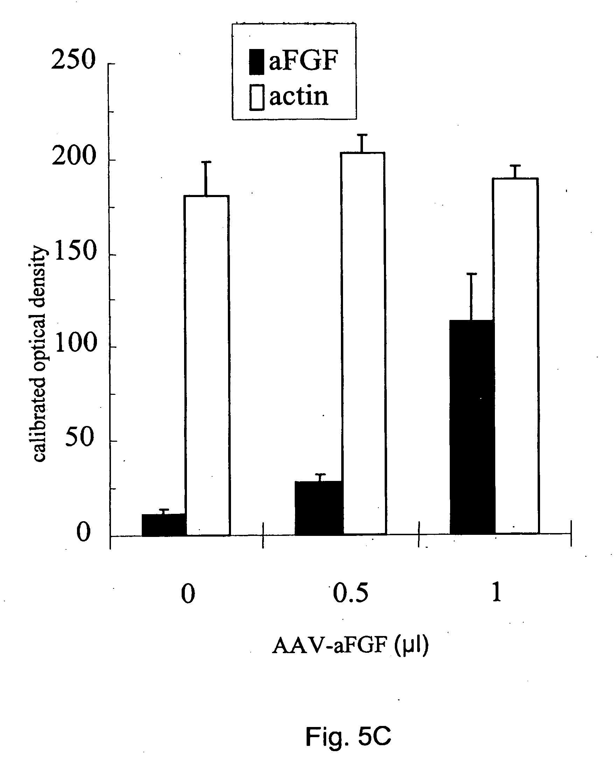

Referring to Fig. 5C, the spinal cord infected with AAV-aFGF showed higher protein expression of aFGF of SEQ ID NO: 2 than the spinal cord not infected with any AAV-aFGF. The protein expression was increased significantly as the spinal cord was infected with 1 µl of AAV-aFGF.

Example 5: Behavioral Assessment

All of the animals received two behavioral tests each week from 1 week until 6 weeks post-surgery. All behavioral tests were videotaped and the two examiners were not aware of which animals were treated when they participated in behavior evaluation. The hind limb locomotor behavior of rats was evaluated by the Basso, Beattie, Bresnahan (BBB) open field locomotor test (Basso et al., 1995 Journal of Neurotrauma 12:1-21) and the combined behavior score (CBS).

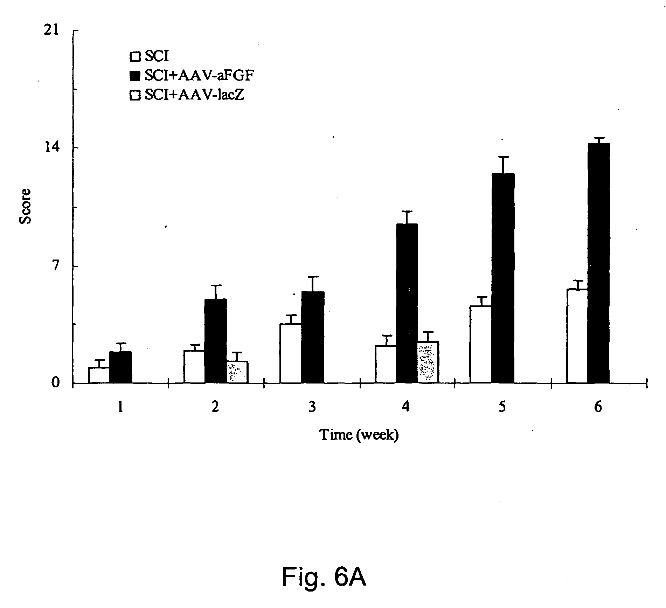

First, the BBB analysis for rats was preformed in an open field (at least 150 cm × 100 cm). The numbers of the contrusive spinal cord injury rats were 8 for the control vehicle treated group (SCI), 12 for the AAV-aFGF treated group (SCI+AAV-aFGF) and 3 for AAV-*lacZ* treated group (SCI+AAV*-lacZ),* respectively. Each session lasted for 5 minutes. The open field locomotor activity score would be determined by observing and scoring the behaviors involving the hip movement, knee movement, and ankle movement, as well as the position between trunk and tail or hind-limb. Detailed estimations on the stepping, toe clearance, paw placement, weight support and coordination were also determined. The scores ranged from 0 to 21 (0 for no movement, and 21 for normal movement). Referring to Fig. 6A, the SCI+AAV-aFGF group has shown a dramatic increase in the score, indicating that the contrusion rats that received the intraspinal injection of AAV-aFGF recovered from the nerve injury much better than the contrusion rats without the injection of AAV-aFGF or the contrusion rats injected with AAV-*lacZ*. The increased score was most significant for the SCI+AAV-aFGF group from the 4^{th} week to the 6^{th} week as compared to the SCI or the SCI-AAV*-lacZ* groups.

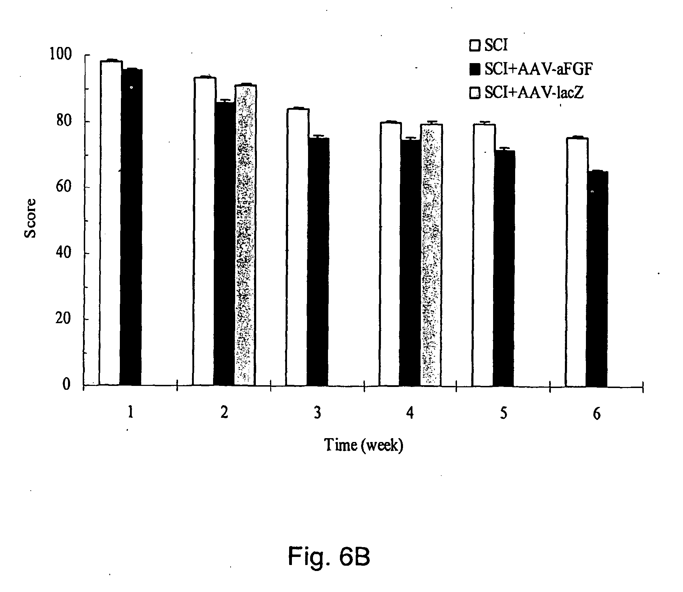

Second, the CBS analysis was a combination test from the Tarlov scoring system test and inclined plane test for evaluating single and complex reflexes, as well as the spontaneous and evoked motor patterns. This multiple measurement contained motor function, toe spread, righting, withdrawal response, placing, inclined plane and swimming tests. The recorded behavior level was graded and scored. The rats which lost most of their locomotor activity were scored as 100, and the rats with the most improvement on the locomotor activity were scored as less than 100 and near to zero point. In Fig. 6B, the CBS score was decreased for the contrusion rats treated with AAV-aFGF as the time elapsed. The CBS score for the contrusion rats treated with AAV-aFGF was also lower than those SCI rats receiving AAV-*lacZ* treatment or the control vehicle. Accordingly, the contrusion rats that received the AAV-aFGF treatment showed the most significant improvement on the locomotor activity.

## Claims

1. Use of a gene coding for an acid fibroblast growth factor (aFGF) for the manufacture of a medicament for treating a nerve injury or regenerating a neuron, wherein the gene is delivered by a viral vector and wherein the viral vector comprises at least one of an adeno-associated virus (AAV) gene and an adenovirus gene.

2. Use according to claim 1, wherein the gene comprises a DNA sequence having at least 70% identity with SEQ ID NO: 1.

3. Use according to claim 1, wherein the gene is a gene coding for an aFGF comprising an amino acid sequence having at least 70% identity with SEQ ID NO: 2.

4. Use according to any one of claims 1 to 3, wherein the vector further comprises a reporter gene.

5. Use according to claim 4, wherein the reporter gene includes a bacterial *lacZ* cDNA.

6. Use according to any one of claims 1 to 5, wherein the injured nerve is at least one of a nerve organ, a nerve tissue, a neuronal cell system transducible with a vector, and a site at which neuronal cells or processes thereof reside.

7. Use according to any one of claims 1 to 5, wherein the neuron includes at least one of an astrocyte, oligodendrocyte, neuroglial cell, choroids plexus cell, ependymal cell, meningeal cell, Schwann cell, fibroblast, microglial cell, a cell in the spinal cord tissue and a cell line of neuronal origin transformed with the gene.

8. A vector construct for treating a nerve injury or regenerating a neuron, wherein the vector comprises a viral vector carrying a gene coding for an acid fibroblast growth factor (aFGF), wherein the viral vector comprises at least one of an adeno-associated virus (AAV) gene and an adenovirus gene.

9. A vector construct according to claim 8, wherein the gene comprises a DNA sequence having at least 70% identity with SEQ ID NO: 1.

10. A vector construct according to claim 8, wherein the gene is a gene coding for an aFGF comprising an amino acid sequence having at least 70% identity with SEQ ID NO: 2.

11. A vector construct according to any one of claims 8 to 10, further comprising:
(a) a viral genome for packaging the viral vector into viral particles; and/or
(b) a reporter gene.

12. A vector construct according to claim 11, wherein the reporter gene is a bacterial *lac*Z cDNA.

## Patentansprüche

1. Verwendung eines Gens, das einen sauren Fibroblastenwachstumsfaktor (aFGF) kodiert, zur Herstellung eines Medikaments für die Behandlung einer Nervenverletzung oder für die Regeneration eines Neurons, wobei das Gen durch einen viralen Vektor zugeführt wird und wobei der virale Vektor mindestens ein Adeno-assoziiertes Virus- (AAV-) Gen und ein Adenovirus-Gen umfasst.

2. Verwendung nach Anspruch 1, wobei das Gen eine DNA-Sequenz mit mindestens 70% Identität mit SEQ ID Nr. 1 umfasst.

3. Verwendung nach Anspruch 1, wobei das Gen ein Gen ist, das einen aFGF kodiert, der eine Aminosäuresequenz mit mindestens 70% Identität mit SEQ ID Nr. 2 umfasst.

4. Verwendung nach einem der Ansprüche 1 bis 3, wobei der Vektor ferner ein Reportergen umfasst.

5. Verwendung nach Anspruch 4, wobei das Reportergen eine bakterielle *lacZ-*cDNA einschließt.

6. Verwendung nach einem der Ansprüche 1 bis 5, wobei der verletzte Nerv mindestens einer eines Nervenorgans, eines Nervengewebes, eines neuronalen Zellsystems, das mit einem Vektor transduzierbar ist, und eine Stelle ist, an welcher sich neuronale Zellen oder Prozesse davon befinden.

7. Verwendung nach einem der Ansprüche 1 bis 5, wobei das Neuron mindestens einen eines Astrozyten, Oligodendrozyten, einer Gliazelle, Aderngeflechtzelle, Ependymzelle, meningealen Zelle, Schwann-Zelle, eines Fibroblasten, einer Mikrogliazelle, einer Zelle im Rückenmarksgewebe und einer Zelllinie neuronalen Ursprungs, die mit dem Gen transformiert ist, einschließt.

8. Vektorkonstrukt zur Behandlung einer Nervenverletzung oder zur Regeneration eines Neurons, wobei der Vektor einen viralen Vektor umfasst, der ein Gen trägt, das einen sauren Fibroblastenwachstumsfaktor (aFGF) kodiert, wobei der virale Vektor mindestens ein Adeno-assoziiertes Virus- (AAV-) Gen und ein Adenovirus-Gen umfasst.

9. Vektorkonstrukt nach Anspruch 8, wobei das Gen eine DNA-Sequenz mit mindestens 70% Identität mit SEQ ID Nr. 1 umfasst.

10. Vektorkonstrukt nach Anspruch 8, wobei das Gen ein Gen ist, das einen aFGF kodiert, der eine Aminosäuresequenz mit mindestens 70% Identität mit SEQ ID Nr. 2 umfasst.

11. Vektorkonstrukt nach einem der Ansprüche 8, bis 10, das ferner umfasst:
(a) ein virales Genom zum Verpacken des viralen Vektors in Virione; und/oder
(b) ein Reporter-Gen.

12. Vektorkonstrukt nach Anspruch 11, wobei das Reporter-Gen eine bakterielle *lacZ*-cDNA ist.

## Revendications

1. Emploi d'un gène codant un facteur aFGF (facteur acide de croissance de fibroblastes) en vue de la fabrication d'un médicament conçu pour le traitement d'une lésion nerveuse ou la régénération d'un neurone, lequel gène est apporté par un vecteur viral et lequel vecteur viral comporte au moins l'un des gènes suivants : un gène de virus AAV (virus adéno-associé) et un gène d'adénovirus.

2. Emploi conforme à la revendication 1, pour lequel le gène comprend une séquence d'ADN qui est, pour au moins 70 %, identique à la Séquence N° 1.

3. Emploi conforme à la revendication 1, pour lequel le gène est un gène codant un facteur aFGF qui comprend une séquence d'acides aminés qui est, pour au moins 70 %, identique à la Séquence N° 2.

4. Emploi conforme à l'une des revendications 1 à 3, pour lequel le vecteur comprend en outre un gène rapporteur.

5. Emploi conforme à la revendication 4, pour lequel le gène rapporteur inclut un ADNc *lacZ* bactérien.

6. Emploi conforme à l'une des revendications 1 à 5, le siège de la lésion nerveuse étant au moins l'un des suivants : un organe nerveux, un tissu nerveux, un système de cellules neuronales transductible à l'aide d'un vecteur, et un site au niveau duquel se trouvent des cellules neuronales ou des prolongements de telles cellules.

7. Emploi conforme à l'une des revendications 1 à 5, le neurone comprenant au moins l'un des suivants : un astrocyte, un oligodendrocyte, une cellule neurogliale, une cellule de plexus choroïde, une cellule épendymaire, une cellule méningienne, une cellule de Schwann, un fibroblaste, une cellule microgliale, une cellule de tissu de moelle épinière, et une lignée de cellules d'origine neuronale, transformée au moyen du gène.

8. Construction à vecteur, conçue pour le traitement d'une lésion nerveuse ou la régénération d'un neurone, dans laquelle le vecteur comprend un vecteur viral qui porte un gène codant un facteur aFGF (facteur acide de croissance de fibroblastes), lequel vecteur viral comporte au moins l'un des gènes suivants : un gène de virus AAV (virus adéno-associé) et un gène d'adénovirus.

9. Construction à vecteur, conforme à la revendication 8, dans laquelle le gène comprend une séquence d'ADN qui est, pour au moins 70 %, identique à la Séquence N° 1.

10. Construction à vecteur, conforme à la revendication 8, dans laquelle le gène est un gène codant un facteur aFGF qui comprend une séquence d'acides aminés qui est, pour au moins 70 %, identique à la Séquence N° 2.

11. Construction à vecteur, conforme à l'une des revendications 8 à 10, qui comprend en outre :
a) un génome viral servant à empaqueter le vecteur viral dans des particules virales ;
b) et/ou un gène rapporteur.

12. Construction à vecteur, conforme à la revendication 11, dans laquelle le gène rapporteur est un ADNc *lacZ* bactérien.


### SEQUENCE LISTING

<110> Cheng, Henrich
<120> A method for treating nerve injury or regenerating neuron and a vector construct for the same
<130> N.98467 SMW
<150> US 11/298,821
   <151> 2005-12-09
<160> 10
   <170> PatentIn version 3.3
<210> 1
   <211> 2357
   <212> DNA
   <213> Homo sapiens
<400> 1
   gagccgggct actctgagaa gaagacacca agtggattct gcttcccctg ggacagcact     60
   gagcgagtgt ggagagaggt acagccctcg gcctacaagc tctttagtct tgaaagcgcc    120
   acaagcagca gctgctgagc catggctgaa ggggaaatca ccaccttcac agccctgacc    180
   gagaagttta atctgcctcc agggaattac aagaagccca aactcctcta ctgtagcaac    240
   gggggccact tcctgaggat ccttccggat ggcacagtgg atgggacaag ggacaggagc    300
   gaccagcaca ttcagctgca gctcagtgcg gaaagcgtgg gggaggtgta tataaagagt    360
   accgagactg gccagtactt ggccatggac accgacgggc ttttatacgg ctcacagaca    420
   ccaaatgagg aatgtttgtt cctggaaagg ctggaggaga accattacaa cacctatata    480
   tccaagaagc atgcagagaa gaattggttt gttggcctca agaagaatgg gagctgcaaa    540
   cgcggtcctc ggactcacta tggccagaaa gcaatcttgt ttctccccct gccagtctct     600
   tctgattaaa gagatctgtt ctgggtgttg accactccag agaagtttcg aggggtcctc    660
   acctggttga cccaaaaatg ttcccttgac cattggctgc gctaaccccc agcccacaga    720
   gcctgaattt gtaagcaact tgcttctaaa tgcccagttc acttctttgc agagcctttt    780
   acccctgcac agtttagaac agagggacca aattgcttct aggagtcaac tggctggcca    840
   gtctgggtct gggtttggat ctccaattgc ctcttgcagg ctgagtccct ccatgcaaaa    900
   gtggggctaa atgaagtgtg ttaaggggtc ggctaagtgg gacattagta actgcacact    960
   atttccctct actgagtaaa ccctatctgt gattccccca aacatctggc atggctccct   1020
   tttgtccttc ctgtgccctg caaatattag caaagaagct tcatgccagg ttaggaaggc 1080
   agcattccat gaccagaaac agggacaaag aaatcccccc ttcagaacag aggcatttaa 1140
   aatggaaaag agagattgga ttttggtggg taacttagaa ggatggcatc tccatgtaga 1200
   ataaatgaag aaagggaggc ccagccgcag gaaggcagaa taaatccttg ggagtcatta 1260
   ccacgccttg accttcccaa ggttactcag cagcagagag ccctgggtga cttcaggtgg 1320
   agagcactag aagtggtttc ctgataacaa gcaaggatat cagagctggg aaattcatgt 1380
   ggatctgggg actgagtgtg ggagtgcaga gaaagaaagg gaaactggct gaggggatac 1440
   cataaaaaga ggatgatttc agaaggagaa ggaaaaagaa agtaatgcca cacattgtgc 1500
   ttggcccctg gtaagcagag gctttggggt cctagcccag tgcttctcca acactgaagt 1560
   gcttgcagat catctgggga cctggtttga atggagattc tgattcagtg ggttgggggc 1620
   agagtttctg cagttccatc aggtcccccc caggtgcagg tgctgacaat actgctgcct 1680
   tacccgccat acattaagga gcagggtcct ggtcctaaag agttattcaa atgaaggtgg 1740
   ttcgacgccc cgaacctcac ctgacctcaa ctaaccctta aaaatgcaca cctcatgagt 1800
   ctacctgagc attcaggcag cactgacaat agttatgcct gtactaagga gcatgatttt 1860
   aagaggcttt ggccaatgcc tataaaatgc ccatttcgaa gatatacaaa aacatacttc 1920
   aaaaatgtta aacccttacc aacagctttt cccaggagac catttgtatt accattactt 1980
   gtataaatac acttcctgct taaacttgac ccaggtggct agcaaattag aaacaccatt 2040
   catctctaac atatgatact gatgccatgt aaaggccttt aataagtcat tgaaatttac 2100
   tgtgagactg tatgttttaa ttgcatttaa aaatatatag cttgaaagca gttaaactga 2160
   ttagtattca ggcactgaga atgatagtaa taggatacaa tgtataagct actcacttat 2220
   ctgatactta tttacctata aaatgagatt tttgttttcc actgtgctat tacaaatttt 2280
   cttttgaaag taggaactct taagcaatgg taattgtgaa taaaaattga tgagagtgtt 2340
   aaaaaaaaaa aaaaaaa 2357
<210> 2
   <211> 155
   <212> PRT
   <213> Homo sapiens
<400> 2
   Met Ala Glu Gly Glu Ile Thr Thr Phe Thr Ala Leu Thr Glu Lys Phe
   1 5 10
   15
   Asn Leu Pro Pro Gly Asn Tyr Lys Lys Pro Lys Leu Leu Tyr Cys Ser
   20 25
   30
   Asn Gly Gly His Phe Leu Arg Ile Leu Pro Asp Gly Thr Val Asp Gly
   35 40
   45
   Thr Arg Asp Arg Ser Asp Gln His Ile Gln Leu Gln Leu Ser Ala Glu
   50 55 60
   Ser Val Gly Glu Val Tyr Ile Lys Ser Thr Glu Thr Gly Gln Tyr Leu
   65 70 75
   80
   Ala Met Asp Thr Asp Gly Leu Leu Tyr Gly Ser Gln Thr Pro Asn Glu
   85 90
   95
   Glu Cys Leu Phe Leu Glu Arg Leu Glu Glu Asn His Tyr Asn Thr Tyr
   100 105
   110
   Ile Ser Lys Lys His Ala Glu Lys Asn Trp Phe Val Gly Leu Lys Lys
   115 120 125
   Asn Gly Ser Cys Lys Arg Gly Pro Arg Thr His Tyr Gly Gln Lys Ala
   130 135 140
   Ile Leu Phe Leu Pro Leu Pro Val Ser Ser Asp
   145 150 155
<210> 3
   <211> 18
   <212> DNA
   <213> Artificial
   <220>
   <223> synthetic
<400> 3
   atggctgaag gggaaatc 18
<210> 4
   <211> 24
   <212> DNA
   <213> Artificial
   <220>
   <223> synthetic
<400> 4
   ttaatcagaa gagactggca gggg 24
<210> 5
   <211> 24
   <212> DNA
   <213> Artificial
   <220>
   <223> synthetic
<400> 5
   atgtcgttta ctttgaccaa caag 24
<210> 6
   <211> 25
   <212> DNA
   <213> Artificial
   <220>
   <223> synthetic
<400> 6
   ctttttttga caccagacca actgg 25
<210> 7
   <211> 21
   <212> DNA
   <213> Artificial
   <220>
   <223> primer sequence
<400> 7
   cgaattcact agtgattatg g 21
<210> 8
   <211> 22
   <212> DNA
   <213> Artificial
   <220>
   <223> primer sequence
<400> 8
   ctgcaggaat tcgattttaa tc 22
<210> 9
   <211> 20
   <212> DNA
   <213> Artificial
   <220>
   <223> primer sequence
<400> 9
   aagatcctga ccgagcgtgg 20
<210> 10
   <211> 20
   <212> DNA
   <213> Artificial
   <220>
   <223> primer sequence
<400> 10
   agcgatgcct gggtacatgg 20